# EUROPEAN PATENT APPLICATION

(11) **EP 1 270 723 A1**
(43) Date of publication of application: **02.01.2003**
(21) Application number: 01870143.3
(22) Date of filing: 27.06.2001
(51) Int. Cl.: C12N 15/12, C07K 14/435, C12N 15/63, C12N 5/10, C12N 5/12, C07K 16/18, A61K 38/17, A61K 39/395

(54) **Anticomplement polyptide from salivary glands of the tick Ixodes ricinus**

(71) Applicant: Henogen S.A., 6041 Gosselies (BE)
(72) Inventor: Daix, Virginie, B-6900 Marche-en-Famenne (BE); Godfroid, Edmond, B-1020 Bruxelles (BE); Pastoret, Paul-Pierre, B-1000 Bruxelles (BE); Bollen, Alex, B-1701 Itterbeek (BE); Vanderplasschen, Alain, B-1480 Tubize (BE)
(74) Representative: Van Malderen, Joelle

(57) **Abstract**

The present invention is related to a new class of polypeptides which present more than 40% homology with the amino-acid sequence SEQ. ID NO.1 obtained from the salivary gland of the tick Ixodus ricinus and to a pharmaceutical composition comprising it for the treatment and/or the prevention of various diseases, especially a disease induced by tick and pathogenous agents transmitted by tick as well as for the treatment and/or the prevention of cardiac related diseases or inflammations, and/or for controlling tick population infesting animals.

## Description

### Field of the invention

The present invention is related to a new anticomplement polypeptide as well as to its use and its preparation process.

### Background of the invention and state of the art

In an immune response, the complement activation leads to the production of inflammatory anaphylatoxins and to the formation of a membrane attack complex leading to the lysis of the invading organism. Endogenous regulators exist to prevent pathology associated with unconfined and inadvertent complement activation.

However, various pathogens have developed biochemical pathways and mechanisms to evade the host complement system.

Ticks are ectoparasites which infest many animals (mammals, birds, reptiles and amphibians) and are present in almost every region of the world. In addition, many of these ticks are vectors of pathogens such as viruses (toghoto virus), bacteria (borrelia burgdorferi, rickettsia spp) or protozoa (trypanosoma sp, babesia sp, theileria sp) that cause host morbidity and in some cases mortality, in particular in humans and livestock animals.

The tick salivary glands play an important role in the accomplishment of the blood meal and the transmission of said pathogens.

The tick is able to feed repeatedly on its natural host and has a salivary anticomplement activity that presumably facilitates feeding.

Valenzuela J. et al. (The Journal of Biological Chemistry, Vol; 275, No.25, p.18717-18723, 2000) describes the purification, cloning and expression of a novel salivary anticomplement protein from the tick Ixodes scapularis.

The authors state that said molecule behaves as a regulator of complement activation in a manner similar to decay accelerating factor and Factor H, which inhibit complement activation by interacting with the C3 convertase of classical or alternative pathways.

However, said sequence does not present any significant sequence similarity with Factor H or with any other protein available in public domain databases. It would thus appear that said molecule belongs to a new class of complement regulatory molecules, which upon successful expression, would constitute appropriate antigens for anti-tick vaccines and/or new therapeutic agents.

### Aims of the invention

The present invention aims to provide new anticomplement polypeptides and polynucleotides encoding them.

A main aim of the present invention is to provide such peptides and polynucleotides which inhibit complement through classical and alternative pathways and which are therefore suitable antigenic candidates for vaccination against ticks and for blocking transmission of various micro-organisms, especially pathogenig agents carried by ticks.

A further aim of the present invention is to provide a pharmaceutical composition which may comprise said anticomplement polypeptide, polynucleotide or variant(s) thereof for modulating the immune response of a mammal, in particular, for reducing complement activation in pathologies involving undesired complement activation such as, for example, post ischemic myocardial destruction and inflammation.

### Summary of the invention

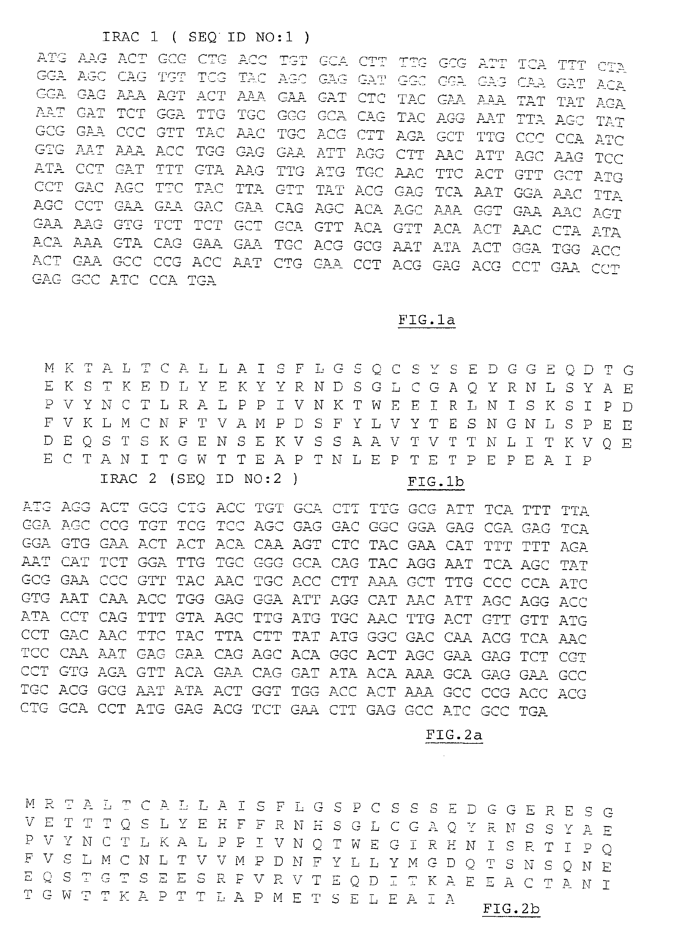

The present invention is related to a novel class of anticomplement compounds presenting an alternative biochemical pathway different from other known anticomplement molecules and which have more than 40%, 50%, 60%, 65%, 70%, 75%, 80%, 85%, 90%, 95% or 98% homology (or sequence identity) with the amino-acid sequence of the polypeptide identified by SEQ ID NO.1 (identified hereafter as molecule IRAC 1) (see Fig. 1).

The present invention is also related to a polynucleotide (or nucleic acid molecules) encoding said polypeptide, preferably a polynucleotide which presents more than 40%, 50%, 60%, 65%, 70%, 75%, 80%, 85%, 90%, 95% or 98% - 99% homology (or sequence identity) with the nucleotide sequence SEQ ID NO.1 or its complementary strand.

Preferably, said polypeptide and its encoding polynucleotide correspond to the complete sequence SEQ ID NO.1 or an active fragment or variant thereof which presents the same or a similar anticomplement activity.

Variants of the polypeptide according to the invention are also molecules which present the same anticomplement activity with one or more genetic modifications (such as deletion or addition of one or more amino-acids) in the complete sequences SEQ ID NO.1, such as naturally occurring allelic variants.

Variants are also molecules which present a similar anti-complement activity to the polypeptides according to the invention through the same biochemical pathway and acting similarly upon the same active site.

Examples of variants of the IRAC1 sequence according to the invention are preferably the polypeptides (and their encoding polynucleotides) SEQ ID NO.2 (identified herafter as molecule IRAC2, see Fig. 2) and the complete sequence of the molecule ISAC1 described by Valenzuala et al. (The Journal of Biological Chemistry, Vol. 275, p.18717-18723, (2000)), incorporated herein by reference.

According to a preferred embodiment of the present invention, said polypeptides are anticomplement polypeptides obtained from ticks of the genus Ixodes (with the provisio that it is not said ISAC polypeptide obtained from the tick Ixodes scapularis), but possibly other polypeptides expressed by tick Ixodes scapularis, but more preferably polypeptides expressed by the tick Ixodes ricinus.

The peptides can be also integrated as "native" protein or are part of a fusion protein or may advantageously include additional amino-acid sequences which contain secretory or leader sequences, prosequences, sequences which elute in purification such as multiple histidinoresidue or an additional sequence for stability during recombinant production.

Said peptides may comprise also marker sequences which facilitate purification of the fuse polypeptide with a sequence as an hexa-histidine peptide as provided in the PQE vector (Invitrogen Inc.) and described by Gentz et al., Proceeding National Academic of Science of the USA, 1989, Vol.86, p.821-824) or an HA tag or glutathione-S transferase. The corresponding polynucleotide may also contain non-coding 5' and 3' sequences such as transcribed non-translated sequences, splicing and polyadenylation signal and ribosome binding sites.

Another aspect of the present invention is related to a vector comprising the polynucleotide or polypeptide according to the invention, said vector being preferably a plasmid, a virus, a liposome or a cationic vesicle able to transfect a cell and to obtain the expression of said polynucleotide by said cell.

A further aspect of the present invention concerns the cell (prokaryotic or eukaryotic cell) transfected by or comprising said vector.

A further aspect of the present invention is related to an inhibitor directed against the polypeptide according to the invention, a fragment (epitope) thereof or a polynucleotide encoding said polypeptide.

Preferably, said inhibitor is an antibody (monoclonal or polyclonal antibody) or an active hypervariable portion thereof. The inhibitor could be also a specific receptor of a blood cell able to interact specifically with said polypeptide or its epitopes. The inhibitor could be also an antisense RNA or a ribozyme directed against the polynucleotide encoding said polypeptide.

The present invention is also related to the cells (hybridomas) expressing and producing said antibody or an active portion thereof.

A further aspect of the present invention is related to a pharmaceutical composition (including a vaccine) comprising an adequate pharmaceutical carrier (or diluent) and at least one of the various elements according to the invention, especially the polypeptide, its variants, its encoding polynucleotide, the vector, the cell transformed by said vector or the inhibitor according to the invention.

Said pharmaceutical composition may comprise also a suitable adjuvant, antioxidant buffer, bacteriostatics and solutions which become biotonic with the blood of the recipient and aqueous and non-aqueous sterile suspensions (which may include suspension agents). The adjuvant used in the pharmaceutical composition is advantageously used for modulating the immune response of a mammal (including a human) in order to improve the characteristic of the pharmaceutical composition according to the invention or to reduce its possible side effects. The suitable pharmaceutical carrier or diluent is selected by the person skilled in the art according to the type of administration to the mammal (oral administration, intravenous administration, intradermal administration, intramuscular administration, peritoneal administration, etc.).

The pharmaceutical composition can be present in a formulation in a unidose or multidose container and may be stored in a freeze dry condition which requires only the addition of a sterile liquid carrier.

Such pharmaceutical carrier could be in solid liquid or gaseous form and the suitable dose of administration and the ratio between the pharmaceutical carrier/active compound, varies according to the number of administration dose(s), the mass of the mammal to be treated and the possible side effects of the compound according to the invention upon said mammal.

The pharmaceutical composition according to the invention could be a therapeutic or prophylactic composition such as a vaccine.

The pharmaceutical composition according to the invention could be a suitable composition for allowing a vaccination against tick or various micro-organisms (viruses, bacteria or protozoa), transmitted by tick.

The present invention is also related to an immunological/vaccine formulation which comprises the polynucleotide according to the invention presented according to the techniques well-known by the person skilled in the art such as the one described by Wolff et al., Science, Vol.247, p.1465-1468, 1999).

The pharmaceutical composition according to the invention could be used for treating or preventing any disease or pathogenesis involving an uncontrolled or undesired activation of the complement, such as cardiac related disease (in particular partial or total Ischemia, phenomenon of tissue destruction following post-infarctus myocarditis or the genesis of renal tissue destruction due to the presence of circulating immuno-complexes). Such pharmaceutical composition could be also used for developing the inhibition of complement activation for the treatment and/or the prevention of (chronic) inflammations, such as chronic rheumatoid arthritis.

The vaccination against tick could be obtained by inducing a high rejection of tick and/or a neutralisation of the protection conferred by tick saliva to vectors transmitted by tick (viruses, bacteria or protozoa).

Another aspect of the present invention is related to a method of treatment or prevention of a disease affecting or supposed to affect a mammal (especially, live stock animals and humans) transmitted by tick, especially Ixodes ricinus, said method comprising the step of administrating to said mammal a sufficient amount of the pharmaceutical composition according to the invention in order to prevent or cure either the transmission of pathogenic agents by tick, especially Ixodes ricinus, or the symptoms of the disease(s) induced by said pathogenous agents (tick, viruses, bacteria or protozea transmitted by the tick).

The present invention is also related to a method of treatment or prevention of cardiac related disease or inflammation comprising the step of administrating to a mammal affected or supposed to be affected by said disease, a sufficient amount of the pharmaceutical composition according to the invention in order to prevent, reduce or cure the symptoms of said disease.

A final aspect of the present invention is related to the use of the pharmaceutical composition according to the invention for the manufacture of a medicament in the treatment and/or the prevention of various diseases, especially diseases induced by tick or pathogenous agents transmitted by tick, especially Ixodes ricinus, as well as for the treatment or the prevention of cardiac related diseases or inflammations.

The present invention is also related to a method for controlling the population of tick especially but not only Ixodes ricinus infesting animals (mammals, birds, reptiles and amphibians) by administrating to said animals a sufficient amount of the pharmaceutical composition according to the invention in order to avoid a blood meal by said tick.

### Definitions

The definitions of the terms a polypeptide, polynucleotide, variant, homology (or sequence identity) are the ones presented in the document WO 00/77198 incorporated herein by reference and presented as illustration of such definitions well known by the person skilled in the art.

It is meant by an anticomplement molecule: a compound which is able to interact with one of the component of the complement system and to block its biological properties.

### Short description of the drawing

The Fig. 1 and 2 represent the nucleotide and amino acid sequences of the IRAC1 and IRAC2 sequences.

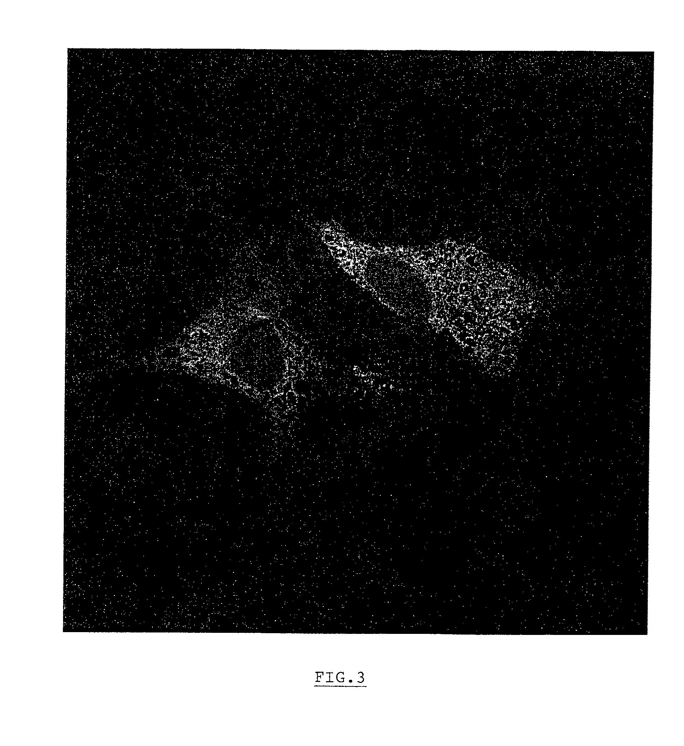

The Fig. 3 represents COS-7 cells transfected by the vector pcDNA3.1 - IRAC 2HA (revelation of Tag HA by indirect immunofluorescent labelling).

### Detailed description of the invention

### 1. Identification of the anticomplement polypeptides according to the invention

mRNAs were extracted from salivary glands of female ticks. After the blood meal, the mRNAs were purified with oligo-dT cellulose (Fasttrack 2.0 kit Invitrogen, Inc.). A reverse transcription has been made with an oligodT primer.

PCR reactions were made with the following forward primers: 5'-acagcactgaggtttcagag-3' and 5'-cagagcaagggtctaccagcc-3' determined upon the base of the published sequence ISAC (Valenzuela et al.) and an oligo-dT reverse primer.

PCR products were cloned into the vector pGEM-TEasy or pGEM-T (Promega), and the inserts were sequenced. This approach allows the recovering of two consensus sequences encoding homologous proteins. These proteins are hereafter identified as IRAC 1 and IRAC 2 for "Ixodes ricinus anticomplement proteins" 1 and 2 respectively. A percentage of identity of said nucleotide and protein sequence of ISAC, IRAC 1 and IRAC 2 is presented in the following table 1.

**Table 1:**

| Identities expressed in percentages of the nucleotide and protein sequences of IRAC and Isac proteins. | | | | |
|---|---|---|---|---|
| | Isac | IRAC1 | IRAC2 | |
| Isac | | 79,8 | 78,9 | DNA |
| IRAC1 | 65,8 | | 81,5 | |
| IRAC2 | 66,3 | 65,2 | | |
| | Protein | | | |

The analysis of the sequences has revealed that IRAC 1 and 2 are original molecules which present a phylogenic analogy with an Isac molecule.

Cloning and expression of the isolated cDNA for IRAC 1 and 2 were achieved by transfection into eukaryotic cells. Corresponding cDNAs were inserted in the vector pcDNA 3.1. Three constrcutions were made (for each cDNA) in order to obtain a non tagged version of the corresponding proteins as well as two additional C-terminal-tagged versions of the proteins (HA or 6xHis). The transfection of COS-7 cells with said constructions has allowed a high expression of the tagged proteins identified by indirect immuno-labelling (see figure 1, a cell expressing the tagged HA IRAC 2 protein).

Since it is possible that the expression of IRAC proteins is toxic to transfected cells, it was proposed to obtain the conditional production of IRAC 1 and IRAC 2 proteins in a cell line (T-Rex System of Invitrogen), after induction by the addition of tetracycline. Four genetic constructions were made using the vector pcDNA4/TO and pcDNA4/TO/myc-His which allow the production of native and tagged proteins (6xHis).

These constructions were transfected into T-Rex-Hela cells (Invitrogen) and stable cell lines were selected with Zeocine.

The tagged proteins were purified thereafter by afinity chromatography upon Ni-chelate columns. Said proteins were thereafter used for animal immunisation in order to produce monoclonal antibodies and polyclonal sera directed against said IRAC 1 and IRAC 2 proteins. These antibodies allow biochemical characterisation of said proteins as well as the identification of cellular and tissular distribution of thesaid proteins.

Such antibodies were used in experimental passive immunity transfer and were thereafter studied for the capacity to inhibit blood meal of ticks and/or transmission of pathogenic agents (virus, bacteria, protoza) to a mammalian host.

The proteins according to the invention were also tested in order to check their binding property to the surface of Borrelia burgdorferi and can therefore confer resistance of said bacteria against deleterious effects of the complement produced by the host.

This phenomenon has already been observed with factor H which binds to the surface protein OspE of Borrelia burgdorferi (Hellwage et al., J.Biol.Chem., Vol.276, p.8427-8435, 2001).

### 2. Inhibition test using classical and alternative methods for the characterisation of complement activity

The proteins according to the invention are thereafter tested for their ability to inhibit complement activity. These tests are called "CH50 Assay for total classical pathway hemolytic activity" and "AH50 Assay for total alternative pathway hemolytic activity". They are based on the protocols described in "Current Protocols in Immunology" (p. 1301-1329, edition 1994).

## Claims

1. Polypeptide which presents more than 40% homology with the amino-acid sequence SEQ.ID.NO.1.

2. Polypeptide according to the claim 1 which presents more than 70% homology with the amino-acid sequence SEQ.ID.NO.1.

3. Polypeptide according to the claim 1 which presents more than 80% homology with the amino-acid sequence SEQ.ID.NO.1.

4. Polypeptide according to the claim 1 which presents more than 90% homology with the amino-acid sequence SEQ.ID.NO.1.

5. Polypeptide according to the claim 1 which presents more than 95% homology with the amino-acid sequence SEQ.ID.NO.1.

6. Polypeptide which presents the amino-acid sequence of SEQ.ID.NO.1, an active fragment thereof or a variant thereof.

7. Polypeptide according to the claim 1 or 2, having the sequence SEQ.ID.NO.2, an active fragment thereof or a variant thereof.

8. Polypeptide according to the claim 1 or 2, having the complete ISAC sequence, an active fragment thereof or a variant thereof.

9. Polypeptide according to any of the preceding claims which presents an anti-complement activity.

10. Polynucleotide encoding the polypeptides according to any of the preceding claims.

11. Vector comprising the polynucleotides or polypeptides according to any of the preceding claims.

12. Cell transfected or comprising the vector according to the claim 11.

13. Inhibitor directed against the polypeptides or the polynucleotides according to any of the preceding claims.

14. Inhibitor according to the claim 13 which is an antibody or an active hypervariable portion thereof.

15. Hybridoma cell expressing the antibody or an active portion thereof according to the claim 14.

16. Pharmaceutical composition comprising an adequate pharmaceutical carrier and an element selected from the group consisting of the polypeptide according to any of the preceding claims 1 to 9, the polynucleotide according to the claim 10, the vector according to the claim 11, the cell according to the claim 12 and/or the inhibitor according to claim 13 or 14 or a mixture thereof.

17. Method for controlling tick population infesting animals, especially but not only Ixodes ricinus, said method comprising the step of administrating to said animals a sufficient amount of the pharmaceutical composition according to the invention in order to prevent animal blood meal by said ticks.

18. Use of the pharmaceutical composition according to the claim 16 for the manufacture of a medicament in the treatment and/or the prevention of diseases induced by tick or pathogenic agents transmitted by ticks, especially but not only Ixodes ricinus.

19. Use of the pharmaceutical composition according to the claim 16 for the manufacture of a medicament in the treatment and/or the prevention of cardiac related diseases or inflammations.


SEQUENCE LISTING

<110>  HENOGEN S.A.

<120>  ANTICOMPLEMENT POLYPEPTIDE

<130>  P.HENO.02/EP

<140>  EP 01870143.3
<141>  2001-06-27

<160>  4

<170>  PatentIn version 3.1

<210>  1
<211>  555
<212>  DNA
<213>  Ixodes ricinus

<220>
<221>  CDS
<222>  (1)..(555)
<223>

<400>  1
atg aag act gcg ctg acc tgt gca ctt ttg gcg att tca ttt cta gga      48
Met Lys Thr Ala Leu Thr Cys Ala Leu Leu Ala Ile Ser Phe Leu Gly
1               5                   10                  15

agc cag tgt tcg tac agc gag gat ggc gga gag caa gat aca gga gag      96
Ser Gln Cys Ser Tyr Ser Glu Asp Gly Gly Glu Gln Asp Thr Gly Glu
            20                  25                  30

aaa agt act aaa gaa gat ctc tac gaa aaa tat tat aga aat gat tct      144
Lys Ser Thr Lys Glu Asp Leu Tyr Glu Lys Tyr Tyr Arg Asn Asp Ser
        35                  40                  45

gga ttg tgc ggg gca cag tac agg aat tta agc tat gcg gaa ccc gtt      192
Gly Leu Cys Gly Ala Gln Tyr Arg Asn Leu Ser Tyr Ala Glu Pro Val
    50                  55                  60

tac aac tgc acg ctt aga gct ttg ccc cca atc gtg aat aaa acc tgg      240
Tyr Asn Cys Thr Leu Arg Ala Leu Pro Pro Ile Val Asn Lys Thr Trp
65                  70                  75                  80

gag gaa att agg ctt aac att agc aag tcc ata cct gat ttt gta aag      288
Glu Glu Ile Arg Leu Asn Ile Ser Lys Ser Ile Pro Asp Phe Val Lys
                85                  90                  95

ttg atg tgc aac ttc act gtt gct atg cct gac agc ttc tac tta gtt      336
Leu Met Cys Asn Phe Thr Val Ala Met Pro Asp Ser Phe Tyr Leu Val
            100                 105                 110

tat acg gag tca aat gga aac tta agc cct gaa gaa gac gaa cag agc      384
Tyr Thr Glu Ser Asn Gly Asn Leu Ser Pro Glu Glu Asp Glu Gln Ser
        115                 120                 125

aca agc aaa ggt gaa aac agt gaa aag gtg tct tct gct gca gtt aca      432
Thr Ser Lys Gly Glu Asn Ser Glu Lys Val Ser Ser Ala Ala Val Thr

130                 135                 140

gtt aca act aac cta ata aca aaa gta cag gaa gaa tgc acg gcg aat       480
Val Thr Thr Asn Leu Ile Thr Lys Val Gln Glu Glu Cys Thr Ala Asn
145                 150                 155                 160

ata act gga tgg acc act gaa gcc ccg acc aat ctg gaa cct acg gag       528
Ile Thr Gly Trp Thr Thr Glu Ala Pro Thr Asn Leu Glu Pro Thr Glu
                165                 170                 175

acg cct gaa cct gag gcc atc cca tga                                   555
Thr Pro Glu Pro Glu Ala Ile Pro
            180

<210>  2
<211>  184
<212>  PRT
<213>  Ixodes ricinus

<400>  2

Met Lys Thr Ala Leu Thr Cys Ala Leu Leu Ala Ile Ser Phe Leu Gly
1               5                   10                  15

Ser Gln Cys Ser Tyr Ser Glu Asp Gly Gly Glu Gln Asp Thr Gly Glu
            20                  25                  30

Lys Ser Thr Lys Glu Asp Leu Tyr Glu Lys Tyr Tyr Arg Asn Asp Ser
        35                  40                  45

Gly Leu Cys Gly Ala Gln Tyr Arg Asn Leu Ser Tyr Ala Glu Pro Val
    50                  55                  60

Tyr Asn Cys Thr Leu Arg Ala Leu Pro Pro Ile Val Asn Lys Thr Trp
65                  70                  75                  80

Glu Glu Ile Arg Leu Asn Ile Ser Lys Ser Ile Pro Asp Phe Val Lys
                85                  90                  95

Leu Met Cys Asn Phe Thr Val Ala Met Pro Asp Ser Phe Tyr Leu Val
            100                 105                 110

Tyr Thr Glu Ser Asn Gly Asn Leu Ser Pro Glu Glu Asp Glu Gln Ser
        115                 120                 125

Thr Ser Lys Gly Glu Asn Ser Glu Lys Val Ser Ser Ala Ala Val Thr
    130                 135                 140

Val Thr Thr Asn Leu Ile Thr Lys Val Gln Glu Glu Cys Thr Ala Asn
145                 150                 155                 160

Ile Thr Gly Trp Thr Thr Glu Ala Pro Thr Asn Leu Glu Pro Thr Glu
                165                 170                 175

Thr Pro Glu Pro Glu Ala Ile Pro
            180

<210>  3
<211>  537
<212>  DNA
<213>  Ixodes ricinus

<220>
<221>  CDS
<222>  (1)..(537)
<223>

<400>  3
atg agg act gcg ctg acc tgt gca ctt ttg gcg att tca ttt tta gga      48
Met Arg Thr Ala Leu Thr Cys Ala Leu Leu Ala Ile Ser Phe Leu Gly
1               5                   10                  15

agc ccg tgt tcg tcc agc gag gac ggc gga gag cga gag tca gga gtg      96
Ser Pro Cys Ser Ser Ser Glu Asp Gly Gly Glu Arg Glu Ser Gly Val
            20                  25                  30

gaa act act aca caa agt ctc tac gaa cat ttt ttt aga aat cat tct      144
Glu Thr Thr Thr Gln Ser Leu Tyr Glu His Phe Phe Arg Asn His Ser
        35                  40                  45

gga ttg tgc ggg gca cag tac agg aat tca agc tat gcg gaa ccc gtt      192
Gly Leu Cys Gly Ala Gln Tyr Arg Asn Ser Ser Tyr Ala Glu Pro Val
    50                  55                  60

tac aac tgc acc ctt aaa gct ttg ccc cca atc gtg aat caa acc tgg      240
Tyr Asn Cys Thr Leu Lys Ala Leu Pro Pro Ile Val Asn Gln Thr Trp
65                  70                  75                  80

gag gga att agg cat aac att agc agg acc ata cct cag ttt gta agc      288
Glu Gly Ile Arg His Asn Ile Ser Arg Thr Ile Pro Gln Phe Val Ser
                85                  90                  95

ttg atg tgc aac ttg act gtt gtt atg cct gac aac ttc tac tta ctt      336
Leu Met Cys Asn Leu Thr Val Val Met Pro Asp Asn Phe Tyr Leu Leu
            100                 105                 110

tat atg ggc gac caa acg tca aac tcc caa aat gag gaa cag agc aca      384
Tyr Met Gly Asp Gln Thr Ser Asn Ser Gln Asn Glu Glu Gln Ser Thr
        115                 120                 125

ggc act agc gaa gag tct cgt cct gtg aga gtt aca gaa cag gat ata      432
Gly Thr Ser Glu Glu Ser Arg Pro Val Arg Val Thr Glu Gln Asp Ile
    130                 135                 140

aca aaa gca gag gaa gcc tgc acg gcg aat ata act ggt tgg acc act      480
Thr Lys Ala Glu Glu Ala Cys Thr Ala Asn Ile Thr Gly Trp Thr Thr
145                 150                 155                 160

aaa gcc ccg acc acg ctg gca cct atg gag acg tct gaa ctt gag gcc 528
Lys Ala Pro Thr Thr Leu Ala Pro Met Glu Thr Ser Glu Leu Glu Ala
                165                 170                 175

atc gcc tga 537
Ile Ala

<210> 4
<211> 178
<212> PRT
<213> Ixodes ricinus

<400> 4

Met Arg Thr Ala Leu Thr Cys Ala Leu Leu Ala Ile Ser Phe Leu Gly
1               5                   10                  15

Ser Pro Cys Ser Ser Ser Glu Asp Gly Gly Glu Arg Glu Ser Gly Val
            20                  25                  30

Glu Thr Thr Thr Gln Ser Leu Tyr Glu His Phe Phe Arg Asn His Ser
        35                  40                  45

Gly Leu Cys Gly Ala Gln Tyr Arg Asn Ser Ser Tyr Ala Glu Pro Val
    50                  55                  60

Tyr Asn Cys Thr Leu Lys Ala Leu Pro Pro Ile Val Asn Gln Thr Trp
65                  70                  75                  80

Glu Gly Ile Arg His Asn Ile Ser Arg Thr Ile Pro Gln Phe Val Ser
                85                  90                  95

Leu Met Cys Asn Leu Thr Val Val Met Pro Asp Asn Phe Tyr Leu Leu
            100                 105                 110

Tyr Met Gly Asp Gln Thr Ser Asn Ser Gln Asn Glu Glu Gln Ser Thr
        115                 120                 125

Gly Thr Ser Glu Glu Ser Arg Pro Val Arg Val Thr Glu Gln Asp Ile
    130                 135                 140

Thr Lys Ala Glu Glu Ala Cys Thr Ala Asn Ile Thr Gly Trp Thr Thr
145                 150                 155                 160

Lys Ala Pro Thr Thr Leu Ala Pro Met Glu Thr Ser Glu Leu Glu Ala
                165                 170                 175

Ile Ala